# EUROPEAN PATENT SPECIFICATION

(11) **EP 4 407 292 B1**
(45) Date of publication and mention of the grant of the patent: **04.02.2026**
(21) Application number: 22915636.9
(22) Date of filing: 02.12.2022
(51) Int. Cl.: G01N 3/00, B23K 11/11, B23K 31/00, G01N 3/08, G01N 3/20, G01N 33/207, B23K 11/16

(54) **METHOD FOR EVALUATING SUSCEPTIBILITY TO LIQUID METAL EMBRITTLEMENT CRACKING IN RESISTANCE SPOT WELDED PORTION OF STEEL SHEET**
VERFAHREN ZUR BEWERTUNG DER ANFÄLLIGKEIT FÜR FLÜSSIGMETALLVERSPRÖDUNGSRISSE IN WIDERSTANDSPUNKTGESCHWEISSTEN TEILEN EINES STAHLBLECHS
ÉVALUATION DE LA SUSCEPTIBILITÉ À LA FISSURATION PAR FRAGILISATION PAR MÉTAL LIQUIDE DANS UNE PARTIE SOUDÉE PAR POINTS PAR RÉSISTANCE D'UNE TÔLE D'ACIER

(30) Priority: 27.12.2021 JP 2021213488; 01.12.2022 JP 2022193165
(43) Date of publication of application: 31.07.2024
(73) Proprietor: JFE Steel Corporation, Tokyo 100-0011 (JP)
(72) Inventor: YASUDA Koichi, Tokyo 100-0004 (JP); IKEDA Rinsei, Tokyo 100-0004 (JP); MIYAKE Ayaka, Tokyo 100-0004 (JP); KAWABE Nao, Tokyo 100-0011 (JP); TAKASHIMA Katsutoshi, Tokyo 100-0011 (JP); OKITA Yasuaki, Tokyo 100-0011 (JP)
(74) Representative: Hoffmann Eitle
(86) International application number: PCT/JP2022/044635
(87) International publication number: WO 2023/127405

(56) References cited:
- CN-A- 112 432 862
- JP-A- 2012 202 947
- JP-A- 2016 045 037
- JP-A- 2020 521 041
- US-A1- 2019 084 074
- FREI JULIAN ET AL: "Investigation of liquid metal embrittlement of dual phase steel joints by electro-thermomechanical spot-welding simulation", vol. 24, no. 7, 1 October 2019 (2019-10-01), GB, pages 624 - 633, XP093232108, ISSN: 1362-1718, Retrieved from the Internet <URL:https://journals.sagepub.com/doi/full-xml/10.1080/13621718.2019.1582203> DOI: 10.1080/13621718.2019.1582203
- SIAR OUTHMANE ET AL: "Impact of liquid metal embrittlement inner cracks on the mechanical behavior of 3 generation advanced high strength steel spot welds", JOURNAL OF MATERIALS RESEARCH AND TECHNOLOGY, vol. 15, 1 November 2021 (2021-11-01), BR, pages 6678 - 6689, XP093232186, ISSN: 2238-7854, DOI: 10.1016/j.jmrt.2021.11.100

## Description

### TECHNICAL FIELD

The present disclosure relates to a method for evaluating susceptibility to liquid metal embrittlement (LME) cracking in a resistance spot welded portion of a steel sheet, and specifically to a method for evaluating susceptibility to LME cracking around the weld metal (nugget) when two or more steel sheets that include a galvanized steel sheet are resistance spot welded. Hereinafter, "susceptibility to LME cracking of a steel sheet" means the ease at which LME cracking occurs as a property specific to a steel sheet.

### BACKGROUND

Since the 1990s, steel sheets subjected to a coating or plating surface treatment including zinc (galvanized steel sheets), such as galvannealed steel sheets, have been widely applied as steel sheets for automotive bodies for the purpose of automobile rust prevention, and many such steel sheets are assembled into automotive bodies by resistance spot welding after blanking and press working.

Recently, as automotive body weight has been reduced to both improve automobile fuel efficiency and meet the demand for improved crashworthiness of automobiles, there has been a trend for galvanized steel sheets to be made stronger. Many high-strength galvanized steel sheets having excellent corrosion resistance have been developed and are widely applied as automotive body components by resistance spot welding.

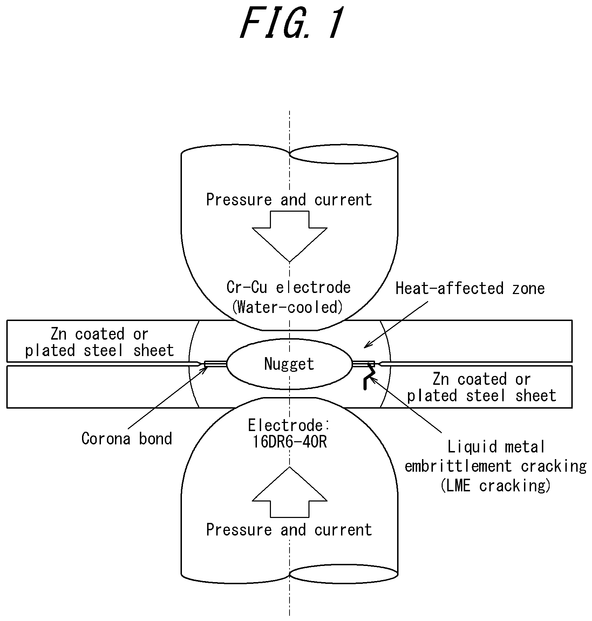

In such resistance spot welding, a sheet combination consisting of two or more overlapping steel sheets is sandwiched between a pair of Cr-Cu electrodes, which are typically water-cooled, and a high current is applied while applying pressure. Joule heating generated at this time melts and solidifies the inside of the sheet combination, forming a nugget of weld metal and joining the sheet combination. Recently, it has become clear that, in the heat-affected zone of resistance spot welding of high-strength galvanized steel sheets, cracking may occur in the thickness direction from the surface of the intersheet gap, as illustrated in FIG. 1, and a cause is liquid metal embrittlement cracking (LME cracking) due to zinc in the coated or plated layer.

Such zinc-induced LME cracking not only reduces welded joint strength, but may also lead to separation of the welded portion on impact and reduced fatigue strength of the welded joint. Accordingly, it is desirable to develop methods to avoid LME cracking occurrence, that is, to apply galvanized steel sheets that have low susceptibility to LME cracking, to develop welding processes, and to develop industrial applications. An important part of the process of such developments is an accurate evaluation of susceptibility to LME cracking.

In the case of industrially applied steel sheets, it is often difficult to evaluate susceptibility to LME cracking with good reproducibility because the frequency of LME cracking occurrence is small in typical resistance spot welding under test conditions (a method in which steel sheets are stacked without gaps and welded with a pair of electrodes positioned vertically relative to the steel sheets). Although there is no systematic and unified method for evaluating LME cracking of such steel sheets, test methods that promote LME cracking occurrence have been empirically applied in various locations, and relative susceptibility to LME cracking evaluated according to the degree of LME cracking occurrence under such conditions.

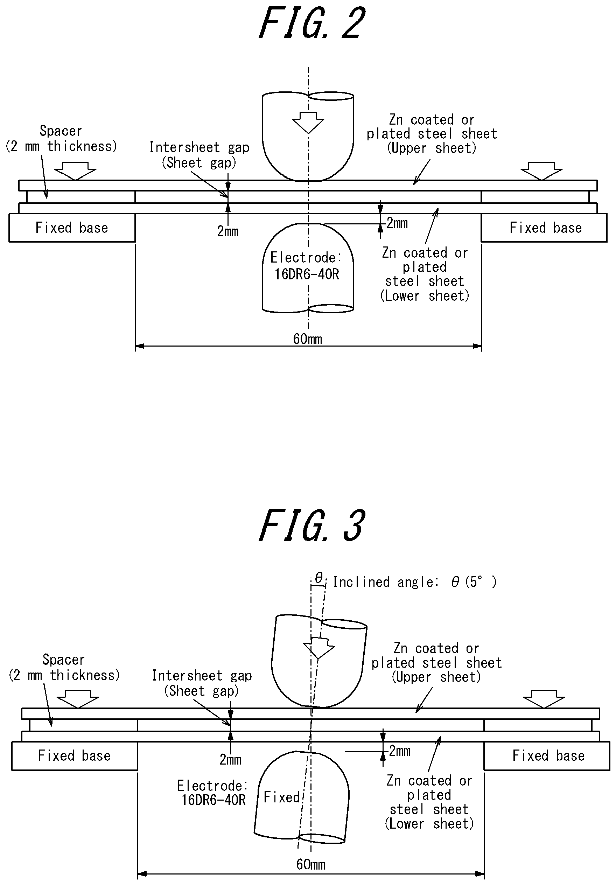

For example, as illustrated in FIG. 2 and FIG. 3, when resistance spot welding two galvanized steel sheets, a sheet gap method is known, in which steel sheets are positioned so that a gap of some millimeters is formed between the sheets (sheet gap) and welded. Further, a sheet gap + electrode inclination angle method is known, in which the electrodes are positioned at an angle misaligned by some degrees from the normal direction of the steel sheets (inclined angle) for pressure application and welding. According to these methods, LME cracking occurrence is promoted by deformation stress and deformation strain generated around the nugget when the applied pressure from the electrodes is released after current application and holding. Susceptibility to LME cracking is then evaluated in a cross-section observation of the welded portion based on presence or absence of cracks, length of cracks, and the like. Non-Patent Literature (NPL) 1 describes the occurrence of LME cracking due to resistance spot welding using a sheet gap + electrode inclination angle method.
NPL 2 describes that a 3D electro-thermomechanical model is established in order to investigate liquid metal embrittlement. After calibration to a dual phase steel of the 1000 MPa tensile strength class, it is used to analyze the thermo-mechanical system of an experimental procedure to enforce liquid metal embrittlement during resistance spot welding. In this procedure, a tensile stress level is applied to zinc coated advanced high strength steel samples during welding. Thereby, liquid metal embrittlement formation is enforced, depending on the applied stress level and the selected material. The model is suitable to determine and visualize the corresponding underlying stresses and strains responsible for the occurrence of liquid metal embrittlement. Simulated local stresses and strains show good conformity with experimentally observed surface crack locations.
NPL 3 describes that a controlled degradation of the welding parameters is used to produce homogeneous advanced high strength steel (AHSS) resistance spot welds with significant liquid metal embrittlement (LME) inner cracks and study their impact on mechanical behavior. The effect of LME inner cracks on damage development is monitored using interrupted cross tension and tensile shear test. Despite their potential effect on the stress state at the notch tip, the observed LME inner cracks seldom contribute to the final fracture path of the welds, leaving the load bearing capacity and failure type of the welds mostly unaffected in tensile shear or even increased in cross tension.

### CITATION LIST

### Non-Patent Literature

NPL 1: Influence of LME-crack generation position on Joint Strength by visualizing method, Preprints of the National Meeting of JWS, Vol. 103 (Sept. 2018).
NPL 2: Frei et al.: "Investigation of liquid embrittlement of dual phase steel joints by electro-thermomechanical spot-welding simulation", Science and Technology of Welding and Joining, Vol. 24, No. 7, 1. Oct. 2019, p. 624-633. NPL 3: Siar et. al: "Impact of liquid metal embrittlement inner cracks on the mechanical behavior of 3rd generation advanced high strength steel spot welds", Journal of Materials Research and Technology, Vol. 15, 1. Nov. 2021, p. 6678-6689.

### SUMMARY

### (Technical Problem)

However, such conventional methods of evaluating susceptibility to LME cracking result in a significant bias in welding current path compared to welding without a sheet gap or inclined angle, and evaluation is made without reproducing a preferred nugget shape. Such bias in current path is affected by sheet gap and inclined angle, as well as by strength level of the steel sheets. Further, deformation stress and deformation strain generated around the nugget when the pressure from the electrodes is released may be expected to vary depending on strength level of the steel sheets. Further, there is a concern that an excessive sheet gap hinders contact between the steel sheets at a site where LME cracking occurs, making it unlikely that molten zinc from the welding is held, which in turn instead makes it more unlikely for LME cracking to occur.

Accordingly, such conventional methods of evaluating susceptibility to LME cracking may be said to have low LME cracking occurrence reproducibility when welding conditions and steel grades are changed, due to instability of welded portion shape, mechanical properties, zinc contact conditions, and the like. Further, such conventional methods of evaluating susceptibility to LME cracking are qualitative or relative evaluations of whether or not LME cracking has occurred, and do not provide a quantitative evaluation of susceptibility, nor do they provide an evaluation of a critical amount of strain at which LME cracking occurs.

In view of the above problems, it would be helpful to provide a method that enables highly accurate and quantitative evaluation of susceptibility to liquid metal embrittlement cracking in a resistance spot welded portion of a steel sheet.

### (Solution to Problem)

The method for evaluating susceptibility to liquid metal embrittlement cracking in a resistance spot welded portion of a steel sheet is defined in claim 1. Distinct embodiments of the methods are defined in the dependent claims. Primary features of the present disclosure are described below.
[1] A method for evaluating susceptibility to liquid metal embrittlement cracking in a resistance spot welded portion of a steel sheet, the method comprising:
   a welding process of overlapping a first steel sheet and a second steel sheet, one or both of which are galvanized steel sheets, without an intervening gap to obtain a sheet combination, sandwiching the sheet combination between a pair of electrodes having an axial direction perpendicular to main surfaces of the first steel sheet and the second steel sheet, and applying current and pressure via the electrodes to perform resistance spot welding;
   a strain applying process of applying one or both of bending strain and tensile strain controlled to a defined amount of strain to a welded portion of the second steel sheet at the same time as or after the end of the current application of the resistance spot welding;
   an observation process of observing the welded portion of the second steel sheet to ascertain the presence or absence of liquid metal embrittlement cracking as an observation result; and
   an evaluation process of evaluating susceptibility to liquid metal embrittlement cracking in the welded portion of the second steel sheet based on the observation result.
[2] The method for evaluating susceptibility to liquid metal embrittlement cracking according to [1], wherein
   a plurality of test pieces taken from the same type of steel sheet are used as the first steel sheet and a plurality of test pieces taken from the same type of steel sheet are used as the second steel sheet, and the series of processes consisting of the welding process, the strain applying process, and the observation process is performed a plurality of times,
   the strain applying process is performed under a plurality of different conditions where one or both of a time at which strain is applied and the defined amount of strain are different, the plurality of different conditions consisting of a first set of conditions where liquid metal embrittlement cracking occurs in the welded portion of the second steel sheet and a second set of conditions where liquid metal embrittlement cracking does not occur in the welded portion of the second steel sheet, and
   the evaluation process comprises:
      identifying a location of occurrence of the liquid metal embrittlement cracking in the welded portion of the second steel sheet in at least one of the first set of conditions;
      determining, among the plurality of conditions, (i) for the first set of conditions, temperature at the time of applying strain at the location of occurrence as a strain application temperature, and (ii) for the second set of conditions, temperature at the time of applying strain at a location corresponding to the location of occurrence as a strain application temperature;
      determining a relationship between the strain application temperature and the defined amount of strain and the presence or absence of liquid metal embrittlement cracking, based on the observation results for the plurality of conditions; and
      evaluating the susceptibility to liquid metal embrittlement cracking in the welded portion of the second steel sheet based on the relationship.
[3] The method for evaluating susceptibility to liquid metal embrittlement cracking according to [2], wherein
   the evaluation process further comprises:
   identifying, from the relationship, a range of amount of strain and temperature where liquid metal embrittlement cracking occurs in the welded portion of the second steel sheet; and
   evaluating the susceptibility to liquid metal embrittlement cracking in the welded portion of the second steel sheet based on the range.
[4] The method for evaluating susceptibility to liquid metal embrittlement cracking according to [3], wherein
   the evaluation process further comprises:
   determining, from the range, the minimum amount of strain at which liquid metal embrittlement cracking occurs in the welded portion of the second steel sheet; and
   evaluating the susceptibility to liquid metal embrittlement cracking in the welded portion of the second steel sheet based on the minimum amount of strain.
[5] The method for evaluating susceptibility to liquid metal embrittlement cracking according to any one of [1] to [4], wherein the welding process performed a plurality of times is performed under the same welding conditions.
[6] The method for evaluating susceptibility to liquid metal embrittlement cracking according to any one of [1] to [5], wherein the bending strain is applied in the strain applying process by applying bending deformation to the second steel sheet so that the second steel sheet has a certain curvature.
[7] The method for evaluating susceptibility to liquid metal embrittlement cracking according to any one of [1] to [6], wherein the tensile strain is applied in the strain applying process by applying tensile deformation to the second steel sheet.
[8] The method for evaluating susceptibility to liquid metal embrittlement cracking according to any one of [1] to [7], wherein the observation process comprises separating the first steel sheet from the second steel sheet and observing the welded portion of the second steel sheet from a main surface side where the second steel sheet was welded to the first steel sheet.

### (Advantageous Effect)

The method for evaluating susceptibility to liquid metal embrittlement cracking in a resistance spot welded portion of a steel sheet allows the susceptibility to liquid metal embrittlement cracking to be evaluated quantitatively and with high accuracy.

### BRIEF DESCRIPTION OF THE DRAWINGS

In the accompanying drawings:
FIG. 1 is a cross-section diagram illustrating location of occurrence of LME cracking in a resistance spot welded portion;
FIG. 2 is a side view diagram illustrating a conventional method for evaluating susceptibility to LME cracking in a resistance spot welded portion (sheet gap method);
FIG. 3 is a side view diagram illustrating a conventional method for evaluating susceptibility to LME cracking in a resistance spot welded portion (sheet gap method + electrode inclination angle method);
FIG. 4 is a side view diagram illustrating the method for evaluating susceptibility to LME cracking in a resistance spot welded portion according to an embodiment of the present disclosure;
FIG. 5 is a side view diagram illustrating an apparatus for applying bending strain for Examples of the present disclosure;
FIG. 6 is a side view diagram and a plan view diagram illustrating shape of a bending block used for the Examples;
FIG. 7 is a side view diagram illustrating an apparatus for simultaneously applying bending strain and tensile strain;
FIG. 8 is a side view diagram illustrating an apparatus for applying tensile strain;
FIG. 9 is an example of a graph illustrating temperature distribution of a resistance spot welded portion at the end of application of current in resistance spot welding in the method for evaluating susceptibility to LME cracking in a resistance spot welded portion according to an embodiment of the present disclosure;
FIG. 10 is an example of a graph illustrating a temperature history from the end of application of current in resistance spot welding at a location of occurrence of LME cracking (0.5 mm from nugget melt line) in the method for evaluating susceptibility to LME cracking in a resistance spot welded portion according to an embodiment of the present disclosure;
FIG. 11 is a graph illustrating a range of amount of strain and temperature where LME cracking occurs in the welded portion of a steel sheet to be evaluated (second steel sheet) (LME cracking strain-temperature range, hereinafter also referred to as LME cracking temperature range (LTR)), according to an embodiment of the present disclosure;
FIG. 12A is a graph illustrating an LME cracking temperature range LTR identified based on a relationship between strain application temperature and amount of strain applied to a welded portion and the presence or absence of LME cracking in a 780 MPa grade GA steel sheet evaluated as an Example;
FIG. 12B is a graph illustrating an LME cracking temperature range LTR identified based on a relationship between strain application temperature and amount of strain applied to a welded portion and the presence or absence of LME cracking in a 980 MPa grade GA steel sheet evaluated as an Example;
FIG. 12C is a graph illustrating an LME cracking temperature range LTR identified based on a relationship between strain application temperature and amount of strain applied to a welded portion and the presence or absence of LME cracking in a 1180 MPa grade GA steel sheet evaluated as an Example; and
FIG. 13 is a diagram illustrating correspondence between an LME cracking occurrence region at an upper surface of a lower sheet b (second steel sheet) and temperature distribution of a resistance spot welded portion during strain application in the method for evaluating susceptibility to LME cracking in a resistance spot welded portion according to an embodiment of the present disclosure.

### DETAILED DESCRIPTION

### (Mechanism of LME cracking occurrence and technical concept of present disclosure)

Due to heat generated during welding, zinc in a coated or plated layer is heated to a molten state, and alloying with the base metal (base steel sheet) progresses (Fe concentration in the coated or plated layer increases). However, during a cooling process after the end of current application, the zinc remains in a molten state in the heat-affected zone around the nugget until a temperature of about 800 °C. Further, it is understood that during the cooling process, tensile strain acts on the heat-affected zone where the zinc is in a molten state due to thermal contraction of the welded portion, and the molten zinc contacts the austenite grain boundaries of the base metal, which were coarsened by the welding, reducing grain boundary strength, resulting in LME cracking.

That is, LME cracking in resistance spot welding is cracking that occurs along the coarse austenite grain boundaries in the heat-affected zone that is heated to the Ac₃ transformation temperature or more by welding. Accordingly, it is clear that LME cracking occurs during the cooling process after the heat-affected zone reaches the peak heating temperature and coarse austenite grains are formed. In resistance spot welding, the welded portion is subjected to pressure and held by the electrodes during and after current application, and the pressure from the electrodes is released after the nugget has completely solidified. However, the peak heating temperature of the heat-affected zone is reached at the end of current application. Therefore, LME cracking may be reproduced by placing an actual heat-affected zone that includes coarsened austenite grain boundaries and molten zinc in a mechanical tension environment during the cooling process after the end of current application.

Considering the thermal cycle, material property changes, and mechanical behavior of such resistance spot welded portions, it may be considered that the LME cracking phenomenon may be faithfully reproduced by applying a mechanical tensile load to the welded portion at the same time as or after the end of resistance spot welding current application.

The present disclosure therefore provides a method for accurately and quantitatively evaluating LME cracking by applying artificially controlled strain around the nugget formed by a typical resistance spot welding method in which steel sheets are stacked without gaps and welded using electrodes arranged perpendicular to the steel sheets.

As practical methods able to apply artificially controllable mechanical tensile loading to a welded portion in a relatively short period of time, one of bending strain, tensile strain, or combined strain of bending strain and tensile strain may be applied. The presence or absence of LME cracking reproduced by this method enables quantitative evaluation of susceptibility to LME cracking.

### (Method for evaluating susceptibility to LME cracking in resistance spot welded portion of steel sheet)

The method for evaluating susceptibility to LME cracking in a resistance spot welded portion of a steel sheet according to an embodiment of the present disclosure includes:
(I) A welding process of overlapping a first steel sheet and a second steel sheet, one or both of which are galvanized steel sheets, without an intervening gap to obtain a sheet combination, sandwiching the sheet combination between a pair of electrodes having an axial direction perpendicular to main surfaces of the first steel sheet and the second steel sheet, and applying current and pressure via the electrodes to perform resistance spot welding;
(II) A strain applying process of applying one or both of bending strain and tensile strain controlled to a defined amount of strain to a welded portion of the second steel sheet at the same time as or after the end of the current application of the resistance spot welding;
(III) An observation process of observing the welded portion of the second steel sheet to ascertain the presence or absence of liquid metal embrittlement cracking as an observation result; and
(IV) An evaluation process of evaluating susceptibility to liquid metal embrittlement cracking in the welded portion of the second steel sheet based on the observation result.

According to the present embodiment, the "second steel sheet" is the steel sheet to be evaluated for susceptibility to LME cracking, and the "first steel sheet" is the mating steel sheet to be welded over the second steel sheet. One or both of the first steel sheet and the second steel sheet may be a galvanized steel sheet. When only one of the first steel sheet and the second steel sheet is a galvanized steel sheet, the other is any steel sheet that is not galvanized. Even when the second steel sheet, the steel sheet to be evaluated, is not galvanized, as long as the first steel sheet, the mating steel sheet, is galvanized, LME cracking may be a problem in the welded portion of the second steel sheet due to the molten zinc generated.

Hereinafter, "steel sheet" means a thin steel sheet that has a sheet thickness of 0.6 mm to 3.2 mm. Further, "galvanized steel sheet" is a steel sheet having a coated or plated layer containing 30 mass% or more zinc on a surface thereof. Examples include a galvannealed steel sheet (GA), a non-alloyed hot-dip galvanized steel sheet (GI), a Zn-Ni plated steel sheet, and the like.

Further, hereinafter, "welded portion" means a region consisting of weld metal (nugget) and the heat-affected zone (HAZ).

### [Welding process (I)]

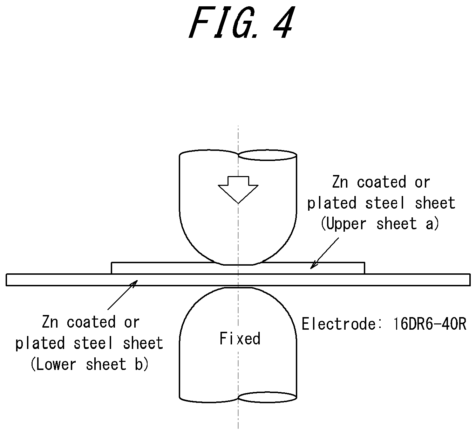

In the welding process (I), as illustrated in FIG. 4, the first steel sheet (upper sheet a as Zn-coated or plated steel sheet in FIG. 4) and the second steel sheet (lower sheet b as Zn-coated or plated steel sheet in FIG. 4) are overlapped without an intervening gap to obtain a sheet combination, the sheet combination is sandwiched by a pair of electrodes having an axial direction perpendicular to main surfaces of the first steel sheet and the second steel sheet, and current and pressure is applied via the electrodes to perform resistance spot welding. By performing resistance spot welding with no sheet gap and no electrode inclined angle, unlike conventional methods, uncontrolled deformation strain is not applied to the welded portion due to pressure applied by the electrode, and this is linked to highly accurate evaluation. The sheet combination may consist of two steel sheets, the first steel sheet and the second steel sheet, or three or more steel sheets including another steel sheet. The first steel sheet and the second steel sheet being overlapped "without a gap" means that there is no spacer between the first steel sheet and the second steel sheet to intentionally create a sheet gap, as illustrated in FIG. 2 and FIG. 3. That is, "without a gap" means that there is no intervening spacer, and it is of course acceptable for micro gaps to exist between the surfaces of both steel sheets. Further, the pair of electrodes having an axial direction "perpendicular" to the main surfaces of the first steel sheet and the second steel sheet means that there is no intentional inclined angle applied to the pair of electrodes, as illustrated in FIG. 3. That is, "perpendicular" does not mean pure mathematical perpendicularity, and inevitable misalignment and error in terms of electrode accuracy are of course acceptable.

### [Strain applying process (II)]

In the strain applying process (II), one or both of bending strain and tensile strain controlled to a defined amount of strain is applied to the welded portion of the second steel sheet at the same time as or after the end of the current application of the resistance spot welding. The timing for applying strain is preferably at the same time as or within a defined time after the end of current application. The "defined time" means a time until the molten zinc from welding re-solidifies.

At such timing, one or both of artificially controlled bending strain and tensile strain is applied to the welded portion of the second steel sheet, which is to be evaluated, such that a mechanical environment that induces cracking is applied to a site most vulnerable to LME cracking around the welded portion. As a result, the evaluation of susceptibility to LME cracking may be performed with high accuracy and excellent reproducibility without being affected by the shape or the like of the nugget or heat-affected zone.

The amount of strain applied to the welded portion of the second steel sheet may be determined in consideration of mechanical properties such as tensile strength of the second steel sheet, chemical composition, and the like, and is not particularly limited. However, for example, an amount of strain of 10 % or less, preferably 5 % or less, may be used to appropriately evaluate susceptibility to LME cracking for any steel grade. A lower limit of the amount of strain is preferably determined according to the minimum amount of strain at which LME cracking occurs in the second steel sheet, and cannot be unconditionally specified, as the lower limit depends on the steel grade, but the amount of strain may be approximately 0.1 % or more.

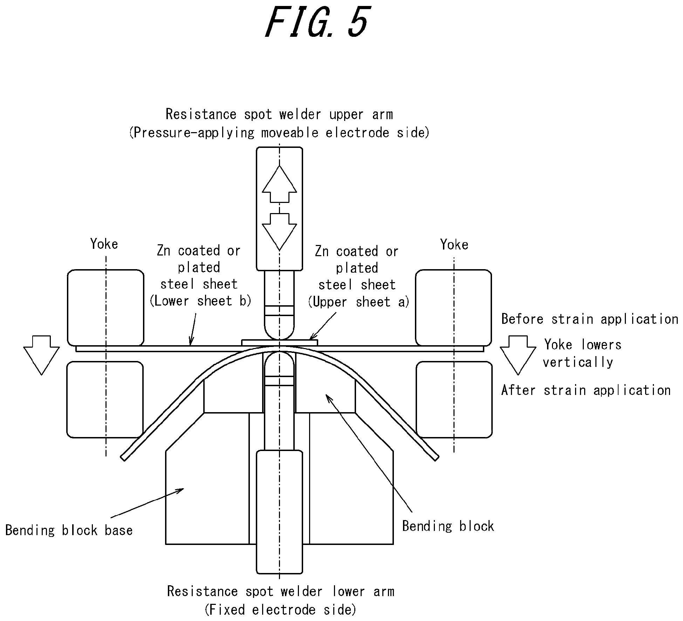

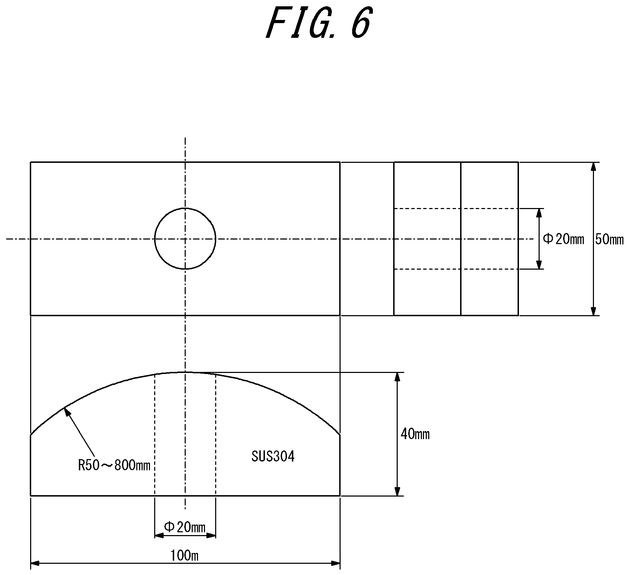

The type of strain applied may be bending strain, tensile strain, or both, as long as the desired amount of strain can be achieved. For example, as illustrated in FIG. 5, bending strain may be applied by applying bending deformation to the second steel sheet (lower sheet b) so that the second steel sheet (lower sheet b) has a certain curvature. In FIG. 5, by lowering the yoke vertically downward, the second steel sheet (lower sheet b) is curved along the bending block that has a surface of a defined curvature, also illustrated in FIG. 6. Accordingly, the second steel sheet (lower sheet b) is curved such that the farther the second steel sheet is from the welded portion, the greater the distance from the first steel sheet (upper sheet a), and a bending strain corresponding to the defined curvature is applied to the welded portion.

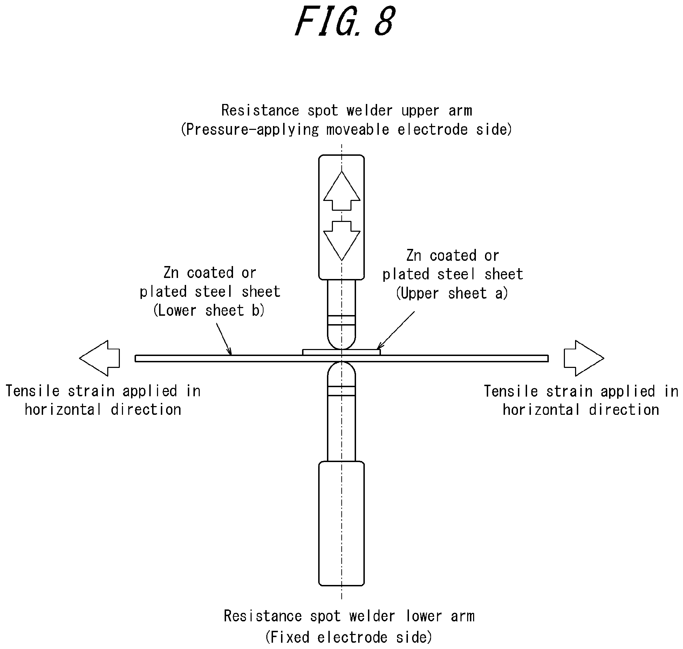

Tensile strain may be applied by applying tensile deformation to the second steel sheet (lower sheet b). For example, as illustrated in FIG. 8, a defined amount of tensile strain is applied to the welded portion by applying a defined tensile deformation in the horizontal direction to the second steel sheet (lower sheet b) installed horizontally.

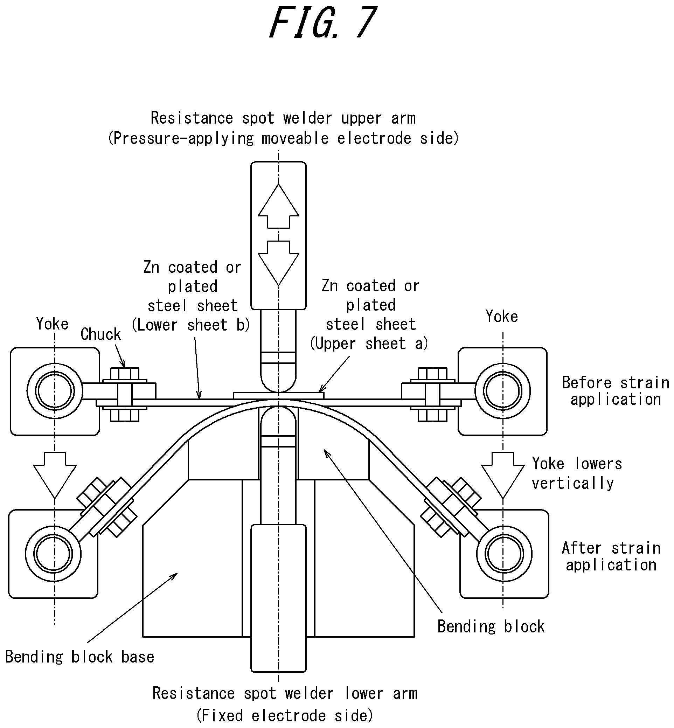

Applying both bending strain and tensile strain may be done, for example, using the apparatus illustrated in FIG. 7. In FIG. 7, the yoke is fixed to the second steel sheet (lower sheet b) by chucks, and by lowering the yoke vertically downward in this state, the second steel sheet (lower sheet b) is curved along the bending block that has a surface of a defined curvature. Accordingly, bending strain corresponding to the defined curvature is applied to the welded portion and the second steel sheet (lower sheet b) is subjected to tensile deformation, and therefore tensile strain of a defined amount of strain is applied to the welded portion.

### [Observation process (III) and evaluation process (IV)]

In the observation process (III), the welded portion of the second steel sheet is observed to ascertain the presence or absence of LME cracking as an observation result. Then, in the evaluation process (IV), the susceptibility to LME cracking in the welded portion of the second steel sheet is evaluated based on the observation result obtained in the observation process (III). According to the present embodiment, susceptibility to LME cracking may be evaluated quantitatively and with high accuracy due to the welding process and the strain applying process described above.

Here, LME cracking is cracking that occurs around the electrodes on the front and back surfaces in contact with the electrodes and cracking that occurs around the nugget at the overlapping surfaces between the steel sheets, which are distinguished as "external cracking" and "internal cracking," respectively. Typically, external cracking occurs at front and back surfaces and is therefore relatively easy to detect and relatively easy to deal with after detection, whereas internal cracking is difficult to detect and has a high risk of remaining in a product.

In the observation process (III), for example, referring to FIG. 1, the first steel sheet (upper sheet a) may be separated from the second steel sheet (lower sheet b) and the welded portion of the second steel sheet (lower sheet b) may be observed from the main surface side that was welded to the first steel sheet (upper sheet a). In this way, internal cracking may be properly observed, which is preferred.

### [Preferred method of evaluating susceptibility to LME cracking]

According to the present embodiment, preferably, test pieces taken from the same type of steel sheet are used as the first steel sheet and test pieces taken from the same type of steel sheet are used as the second steel sheet, and the series of processes consisting of the welding process, the strain applying process, and the observation process is performed a plurality of times. Further, preferably, the strain applying process is performed under a plurality of different conditions where one or both of a time at which strain is applied and the defined amount of strain are different. Here, "same type of steel sheet" means steel sheets that have the same chemical composition, are obtained through a production process under the same conditions, and are expected to have the same specifications as a product and equivalent susceptibility to LME cracking. General examples of "same type of steel sheet" are identical steel sheets and identical coils.

Whether or not LME cracking occurs in test pieces taken from the same type of steel sheet (second steel sheet) depends on the time at which strain is applied (strain application timing) and the amount of strain. According to the present embodiment, the plurality of conditions consists of a first set of conditions where LME cracking occurs in the welded portion of the second steel sheet and a second set of conditions where LME cracking does not occur in the welded portion of the second steel sheet.

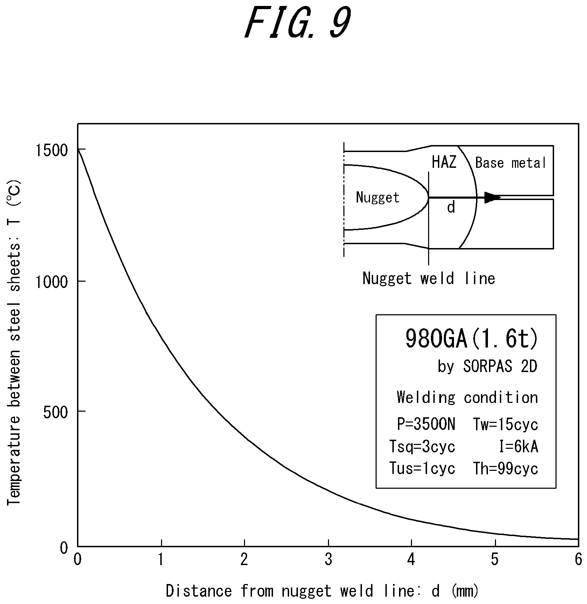

The significance of conducting the series of processes (tests) consisting of the welding process, the strain applying process, and the observation process a plurality of times under a plurality of conditions is explained below. As a result of extensive studies by the inventors, the following discoveries were made. When a typical thin steel sheet for automobiles is used as the second steel sheet and resistance spot welding is performed under typical conditions, the temperature distribution in the resistance spot welded portion at the end of the current application of the resistance spot welding is, as illustrated in FIG. 9 for example, 1500 °C to 800 °C in the heat-affected zone within just 1 mm from the nugget melting line. Under typical conditions, the weld time is 10/50 s to 20/50 s (200 ms to 400 ms), and the temperature distribution in the heat-affected zone immediately after current application is very steep.

In this case, it was found that a location where LME cracking occurs when strain is applied under the first set of conditions is always the same location (for example, about 0.5 mm from the nugget melt line). The region where LME cracking is likely to occur is assumed to be the ring-shaped region between 0.5 mm and 1.0 mm from the nugget melt line, for example. However, when a typical thin steel sheet for automobiles is used and resistance spot welding is performed under typical conditions, multiple cracks do not occur within this small region having a width of just 0.5 mm. This is because cracking occurs only at a site where LME cracking is most likely to occur in the region, and the opening of a crack absorbs and relaxes the surrounding strain. Within this region, the site where LME cracking is most likely to occur is where the austenite grain size of the base metal is largest, that is, the site closest to the nugget melt line (about 0.5 mm from the nugget melt line).

That is, the evaluation process (IV) identifies the location of occurrence of LME cracking in the welded portion of the second steel sheet in at least one of the first set of conditions. The location of occurrence of LME cracking is always the same, as described above, and therefore it is sufficient to identify the location of occurrence of LME cracking in at least one of the first set of conditions.

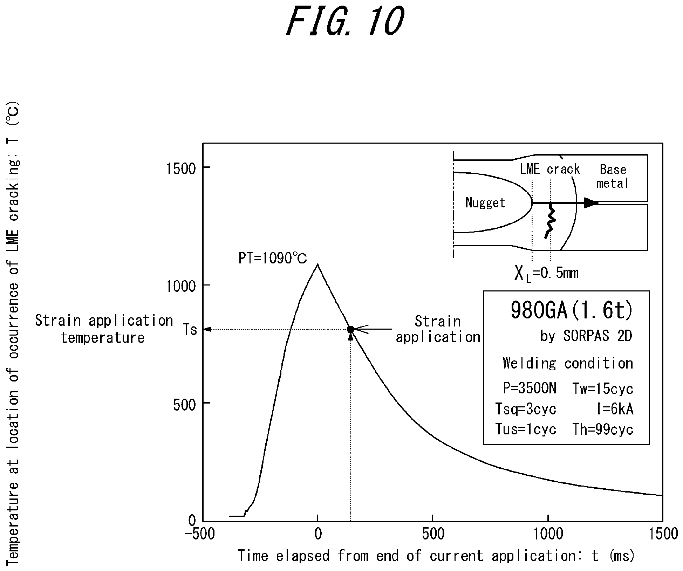

A temperature history from the end of application of current in resistance spot welding at the location of occurrence of LME cracking (0.5 mm from the nugget melt line) when the first set of conditions is used is illustrated in FIG. 10, for example. The location reaches the peak heating temperature (1100 °C, see also FIG. 9) at the end of current application and then cools rapidly. That is, changing the strain application timing within the range of "at the same time as or after the end of the current application of the resistance spot welding" means changing the temperature of the location at the time of strain application.

Therefore, in the evaluation process (IV), among the plurality of conditions, (i) for the first set of conditions, temperature at the time of applying strain at the location of occurrence of LME cracking is determined as a strain application temperature, and (ii) for the second set of conditions, temperature at the time of applying strain at a location corresponding to the location of occurrence of LME (same as for the first set of conditions, at about 0.5 from the nugget melt line, for example) is determined as a strain application temperature.

The temperature distribution of the resistance spot welded portion at a certain time (at the end of current application in FIG. 9) and the temperature history of the resistance spot welded portion from the end of current application in resistance spot welding at a certain location (about 0.5 mm from the nugget melting line in FIG. 10) may be obtained by actual measurement or by numerical analysis using resistance spot welding temperature distribution analysis software.

In the evaluation process (IV), a relationship between the strain application temperature and the defined amount of strain and the presence or absence of LME cracking is determined based on the observation results (presence or absence of LME cracking) for the plurality of conditions, and based on this relationship, the susceptibility to LME cracking in the welded portion of the second steel sheet is evaluated.

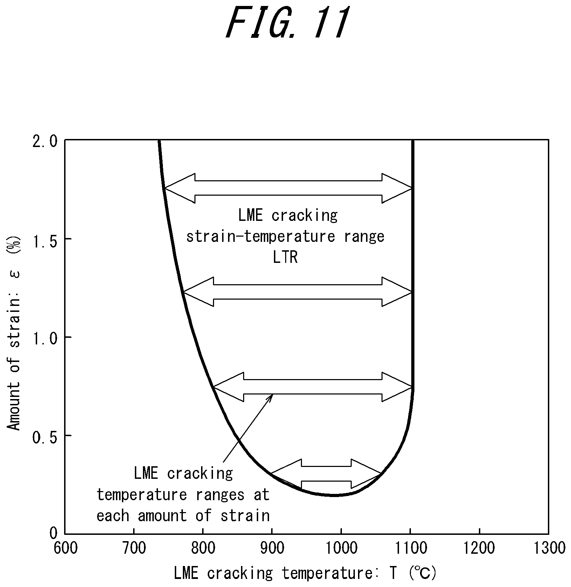

For example, in the strain applying process, the amount of strain to be applied is fixed at a certain value, the timing of strain application is changed in various ways, and multiple tests are conducted to determine the temperature range where LME cracking occurs at each amount of strain (see FIG. 11). For example, it may be evaluated that the narrower the LME cracking temperature range at an amount of strain, the lower the susceptibility to LME cracking. In this example, susceptibility to LME cracking may be evaluated based on the quantitative parameter of the LME cracking temperature range.

By conducting the same test with various amounts of strain, a range of amount of strain and temperature where LME cracking occurs in the welded portion of the second steel sheet (LME cracking temperature range (LTR)) may be determined, as illustrated in FIG. 11. Thus, in the evaluation process (IV), it is preferred that, based on the relationship between the strain application temperature and the defined amount of strain and the presence or absence of LME cracking, the range of amount of strain and temperature where LME cracking occurs (LTR) in the welded portion of the second steel sheet is identified, and the susceptibility to LME cracking in the welded portion of the second steel sheet is evaluated based on this range.

Specifically, from the LTR, the minimum amount of strain (critical amount of strain) at which LME cracking occurs in the welded portion of the second steel sheet is determined, and based on this minimum amount of strain, the susceptibility to LME cracking in the welded portion of the second steel sheet may be evaluated. Specifically, it may be evaluated that the higher the critical amount of strain, the lower the susceptibility to LME cracking. In this example, susceptibility to LME cracking may be evaluated based on a quantitative parameter, the critical amount of strain. Further, the temperature range where LME cracking occurs may be quantitatively ascertained, and the susceptibility to LME cracking of the second steel sheet may be comprehensively evaluated.

When a series of processes consisting of the welding process, the strain applying process, and the observation process is performed a plurality of times, the welding process is preferably performed under the same welding conditions each time. However, in the example of determining the LME cracking strain-temperature range LTR illustrated in FIG. 11, using the same welding conditions is not required. In this case, the same LTR may be obtained even when different welding conditions are used. Therefore, the susceptibility to LME cracking may be properly evaluated by testing under different welding conditions.

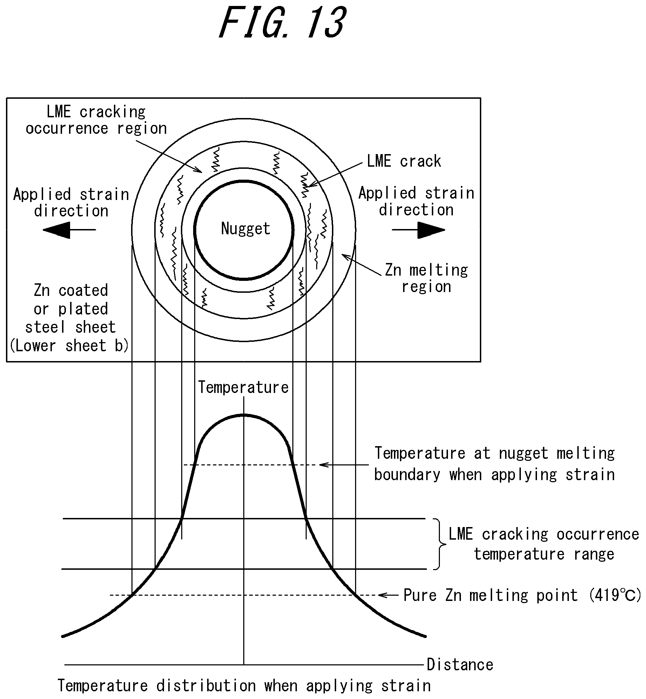

As already mentioned, although not applicable to resistance spot welding under typical conditions for thin steel sheets for automobiles, the following evaluation method may be used when intentionally adopting welding conditions that are not strictly controlled. That is, in the observation process (III), the width of the LME cracking occurrence region concentrically around the nugget in the welded portion of the second steel sheet (lower sheet b) may be determined with reference to the upper illustration in FIG. 13, and the width of the LME cracking occurrence region may be used as an observation result in the evaluation process (IV). For example, it may be evaluated that the smaller the width of the LME cracking occurrence region, the lower the susceptibility to LME cracking. The "LME cracking occurrence region" may be specified as the smallest ring-shaped region that encompasses all LME cracks occurring around the nugget. When intentionally adopting welding conditions that are not strictly controlled, multiple LME cracks occur in the region where LME cracks are likely to occur (for example, the ring-shaped region between 0.5 mm and 1.0 mm from the nugget melt line), and therefore the range where LME cracks occur can be identified as a "region".

A temperature distribution that varies over time is formed around the welded portion due to weld heating, and the temperature distribution at the instant when strain is applied may be determined with relatively high accuracy by actual measurement or numerical analysis. Therefore, determining the temperature distribution of the welded portion of the second steel sheet (lower sheet b) at the time when strain is applied is preferred, as illustrated in the lower illustration of FIG. 13, for example.

The temperature range corresponding to the LME cracking occurrence region (LME cracking temperature range) may be determined by applying the LME cracking occurrence region obtained from the upper illustration of FIG. 13 to the temperature distribution, and the LME cracking temperature range may be used as an observation result in the evaluation process (IV). For example, it may be evaluated that the narrower the LME cracking temperature range, the lower the susceptibility to LME cracking.

In this case, preferably, test pieces taken from the same type of steel sheet are used as the first steel sheet and test pieces taken from the same type of steel sheet are used as the second steel sheet, and the series of processes consisting of the welding process, the strain applying process, and the observation process is performed a plurality of times. Further, preferably, the strain applying process is performed under a plurality of different conditions where the defined amount of strain is different.

Then, in the evaluation process (IV), the susceptibility to LME cracking in the welded portion of the second steel sheet may be evaluated based on the observation results for each of the plurality of conditions. For example, the minimum amount of strain at which LME cracking occurs in the second steel sheet may be determined from the observation results for each of the plurality of conditions, and the susceptibility to LME cracking in the welded portion of the second steel sheet may be evaluated based on the minimum amount of strain (critical amount of strain). Below are two specific methods for determining the critical amount of strain.

A first example is to identify the LME cracking occurrence region as illustrated in the upper illustration of FIG. 13, and then apply the LME cracking occurrence region to the temperature distribution in the lower illustration of FIG. 13 to determine the temperature range corresponding to the LME cracking occurrence region (LME cracking temperature range) for each of the plurality of conditions. Accordingly, the relationship between the amount of strain applied and the LME cracking temperature range (LTR as illustrated in FIG. 11) may be determined. From this relationship, the minimum amount of strain at which LME cracking occurs in the second steel sheet may be determined, and the susceptibility to LME cracking in the welded portion of the second steel sheet may be evaluated based on the minimum amount of strain (critical amount of strain). Specifically, it may be evaluated that the higher the critical amount of strain, the lower the susceptibility to LME cracking. In this example, susceptibility to LME cracking may be evaluated based on a quantitative parameter, the critical amount of strain. Further, the temperature range where LME cracking occurs may be quantitatively ascertained, and the susceptibility to LME cracking of the second steel sheet may be comprehensively evaluated.

Unlike the first example, a second example is evaluation based on the width of the LME cracking occurrence region without identifying the LME cracking temperature range from the LME cracking occurrence region. That is, in the observation process, the width of the LME cracking occurrence region in the welded portion of the second steel sheet is determined for each of the plurality of conditions. Accordingly, the relationship between the amount of strain applied and the width of the LME cracking occurrence region may be determined. From this relationship, the minimum amount of strain at which LME cracking occurs in the second steel sheet may be determined, and the susceptibility to LME cracking in the welded portion of the second steel sheet may be evaluated based on the minimum amount of strain (critical amount of strain). As in the first example, specifically, it may be evaluated that the higher the critical amount of strain, the lower the susceptibility to LME cracking. In this example, susceptibility to LME cracking may be evaluated based on a quantitative parameter, the critical amount of strain.

When a series of processes consisting of the welding process, the strain applying process, and the observation process is performed a plurality of times, the welding process is preferably performed under the same welding conditions each time. However, according to the first example, using the same welding conditions each time is not required. According to the first example, the same LTR may be obtained even when different welding conditions are used. Therefore, the susceptibility to LME cracking may be properly evaluated by testing under different welding conditions.

According to the embodiment described above, the LME cracking strain-temperature range (LTR) and the critical amount of strain obtained from the LTR are indices specific to the steel sheet, including the coated or plated layer, and are also indices that indicate phenomena caused by the metallurgical property behavior of the steel sheet during the thermal cycle process of resistance spot welding. Therefore, the present embodiment is not only a quantitative method for evaluating susceptibility to LME cracking, but may also be applied to the development of materials for the suppression or prevention of LME cracking.

For example, by combining the LTR with the mechanical strain history and thermal hysteresis of the welded portion during the resistance spot welding process of an actual component, the occurrence of LME cracking in the welded portion of the actual component may be predicted and estimated. Application to LME cracking risk management of actual components is also possible, using a margin for the critical amount of strain for cracking.

### EXAMPLES

### (Examples 1 to 3)

In Example 1, 780 MPa grade galvannealed steel sheets (GA steel sheet) each having a thickness of 1.6 mm and a zinc coating weight of 60 g/m² on each side were used as the first steel sheet and the second steel sheet, and resistance spot welding was performed using a single-phase alternating current (AC) welder. In Example 2, 980 MPa grade galvannealed steel sheets (GA steel sheet) each having a thickness of 1.6 mm and a zinc coating weight of 60 g/m² on each side were used as the first steel sheet and the second steel sheet, and resistance spot welding was performed using a single-phase alternating current (AC) welder. In Example 3, 1180 MPa grade galvannealed steel sheets (GA steel sheet) each having a thickness of 1.6 mm and a zinc coating weight of 60 g/m² on each side were used as the first steel sheet and the second steel sheet, and resistance spot welding was performed using a single-phase alternating current (AC) welder. For all of the Examples 1 to 3, 16DR6-40R (DR-type electrode, outer diameter: 16 mm, electrode face: 6 mm diameter, 40R) cap tip electrodes made of Cr-Cu were used as the electrodes for welding, two GA steel sheets were stacked on top of each other, and resistance spot welding was performed with an electrode force of 350 N, a squeeze time of 5/50 s, a weld time of 12/50 s, a hold time of 5/50 s, and a welding current resulting in a nugget diameter of 4√t (= 5.1 mm, t: sheet thickness).

FIG. 5 illustrates a bending strain application apparatus created in implementing Examples 1-3. Six types of bending blocks having bending radii of 50 mm, 70 mm, 120 mm, 200 mm, 400 mm, and 800 mm, respectively, and the shape illustrated in FIG. 6 were used in the bending strain application apparatus. The sheet thickness (t mm) of the steel sheets tested for Examples 1 to 3 was 1.6 mm, and therefore when bending strain was applied to these bending blocks (bending radius: R mm), the amount of strain (ε) on the evaluated steel sheet surface could be obtained as ε = t/(2R + t), which is 1.57 %, 1.13 %, 0.66 %, 0.40 %, 0.20 %, and 0.10 %, respectively. A central portion of these bending blocks had a machined hole through which the electrode for resistance spot welding was disposed without contact. The bending blocks were made from austenitic stainless steel, a non-magnetic material, to avoid impedance loading during welding current application. The power source for applying strain was a hydraulic unit having an operation speed (yoke lowering speed and tensile speed) of 400 mm/s. For controlling the timing of strain application, the displacement of the yoke or tensile crosshead for applying strain was measured using a laser displacement meter and recorded in a data logger at the same time as the welding current waveform was measured, to precisely measure the timing when strain was applied to the welded portion.

For LME cracking evaluation, after welding and applying strain, a 5 mm diameter flat drill was used to drill a hole in only the upper sheet from an upper sheet portion of the welded portion to remove only the upper sheet and expose the upper surface of the lower sheet without scratching or damaging the surface of the lower sheet. The surface was then observed in plan view using a microscope to check for presence or absence of LME cracking and to identify the location of occurrence of any cracks. As a result, it was confirmed that in all of the Examples 1 to 3, under conditions where LME cracking occurred, LME cracking occurred in the heat-affected zone at 0.5 mm from the nugget melt line.

For temperature analysis of the welded portion, SORPAS 2D, a general-purpose software for temperature distribution analysis of resistance spot welding, was used for numerical analysis of the temperature history in the cooling process at a location 0.5 mm from the nugget melt line. An example is illustrated in FIG. 10. For Examples 1 to 3, the temperature at the time of strain application was determined from the temperature history at 0.5 mm from the nugget melt line, and was used as the "strain application temperature". In the above procedure, tensile strain was applied under the above six conditions at the same time as the end of current application and at multiple subsequent timings, and the occurrence of LME cracking was investigated in each test. Based on the results of this study, the susceptibility to LME cracking was comprehensively evaluated by drawing the LME cracking strain-temperature ranges for each of the Examples 1 to 3.

### (Comparative Examples)

As Comparative Examples, conventional LME cracking evaluation methods were performed. That is, three evaluation methods were implemented: a typical welding method, a sheet gap method with a 2 mm gap between sheets, and a sheet gap + electrode inclination angle method with a 5° electrode inclined angle in addition to a 2 mm gap between sheets. For LME cracking evaluation of the Comparative Examples, a welded joint cross section was cut after welding was completed, embedded and polished, etched, then cross-section microstructure observation was performed under a microscope to check and evaluate cracking occurrence. 780 MPa, 980 MPa, and 1180 MPa grade galvannealed steel sheets (GA sheets) having a thickness of 1.6 mm and a zinc coating weight of 60 g/m² on each side were used as the first steel sheet and the second steel sheet, and resistance spot welding was performed using a single-phase AC welder. 16DR6-40R (DR-type electrode, outer diameter: 16 mm, electrode face: 6 mm diameter, 40R) cap tip electrodes made of Cr-Cu were used as the electrodes for welding, two GA steel sheets of the same steel grade were stacked on top of each other, and resistance spot welding was performed with an electrode force of 350 N, a squeeze time of 5/50 s, a weld time of 12/50 s, a hold time of 5/50 s, and a welding current resulting in a nugget diameter of 4√t (= 5.1 mm, t: sheet thickness).

### [Evaluation results]

Tables 1 to 4 summarize the evaluation results for susceptibility to LME cracking of 780 MPa, 980 MPa, and 1180 MPa grade GA steel sheets, where Table 1 lists evaluation results for the Comparative Examples and Tables 2 to 4 list evaluation results for the Examples.

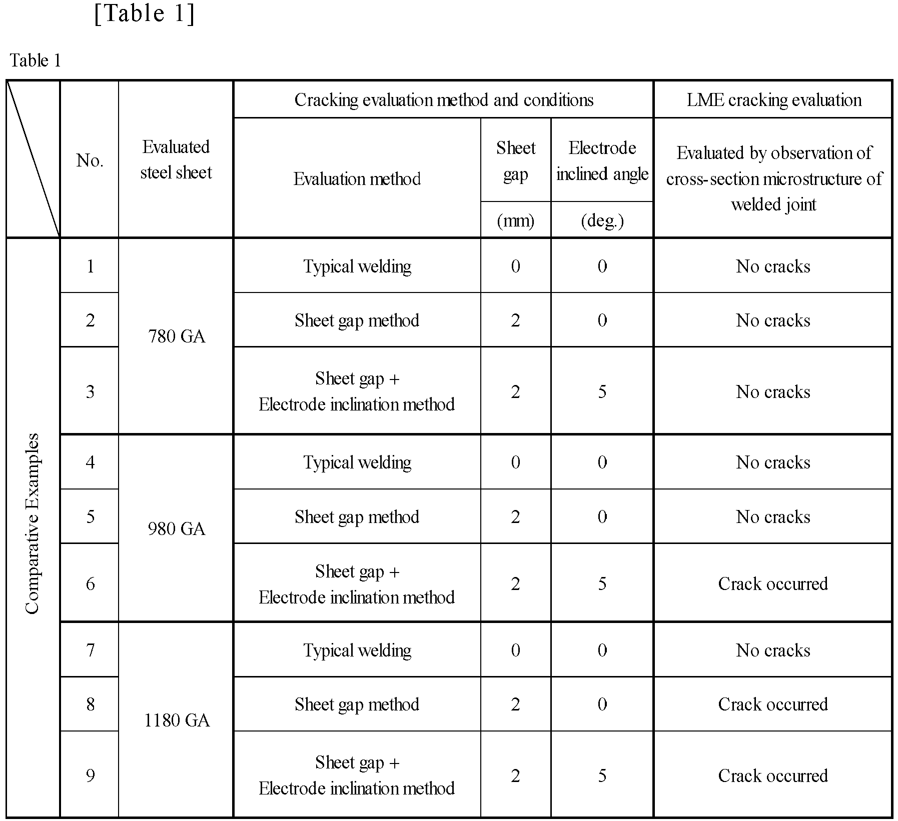

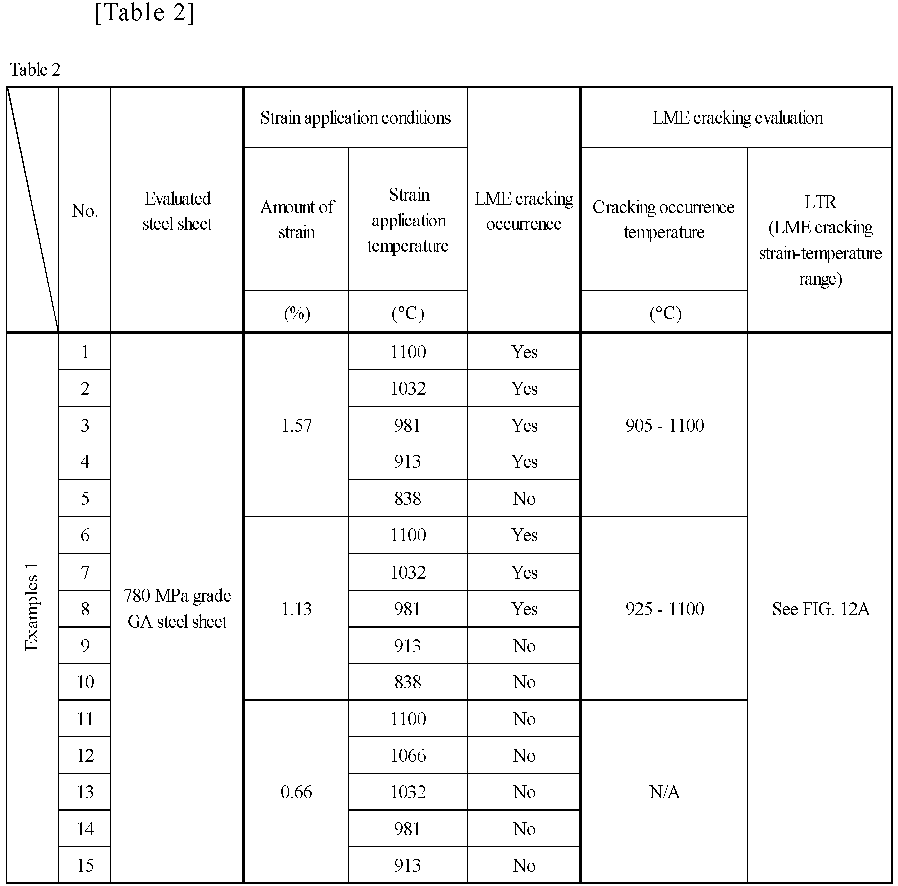

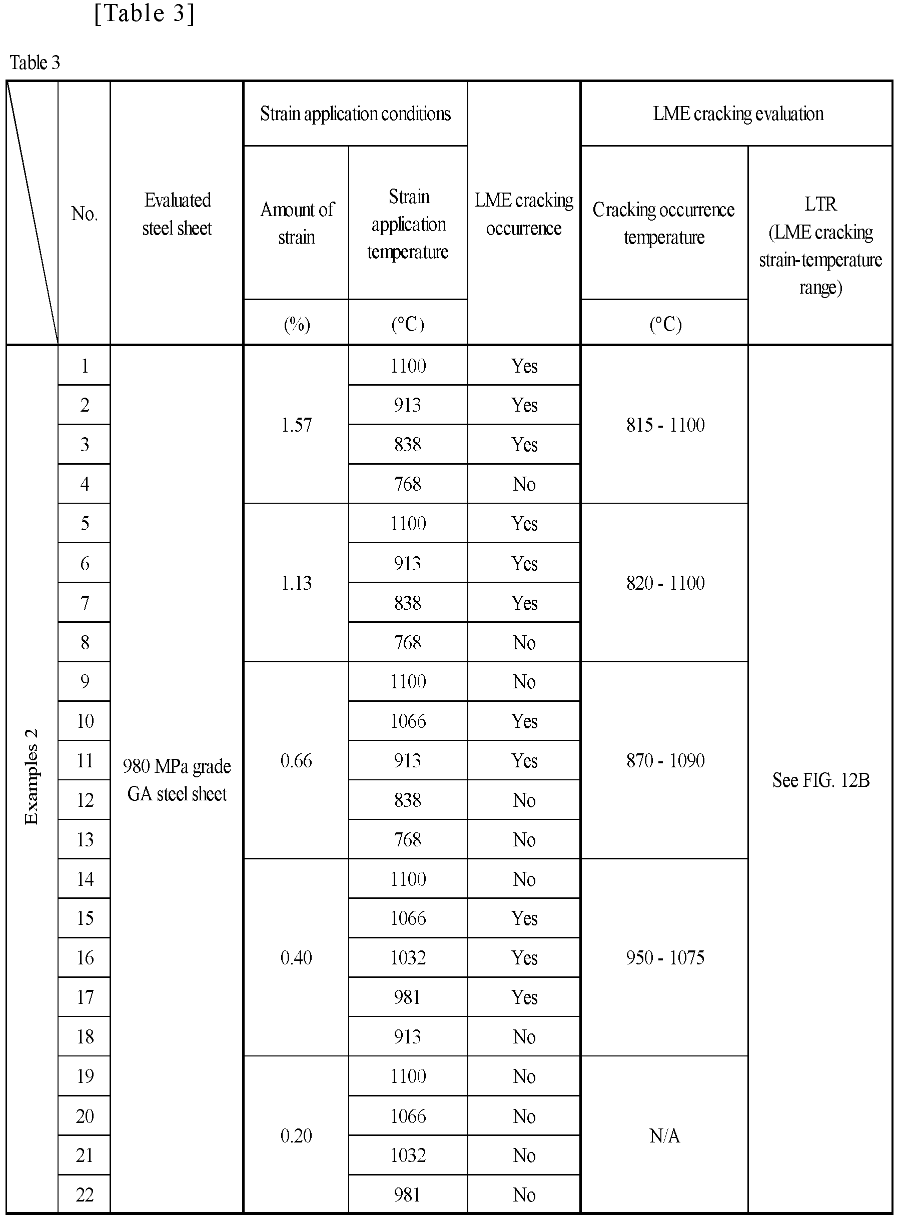

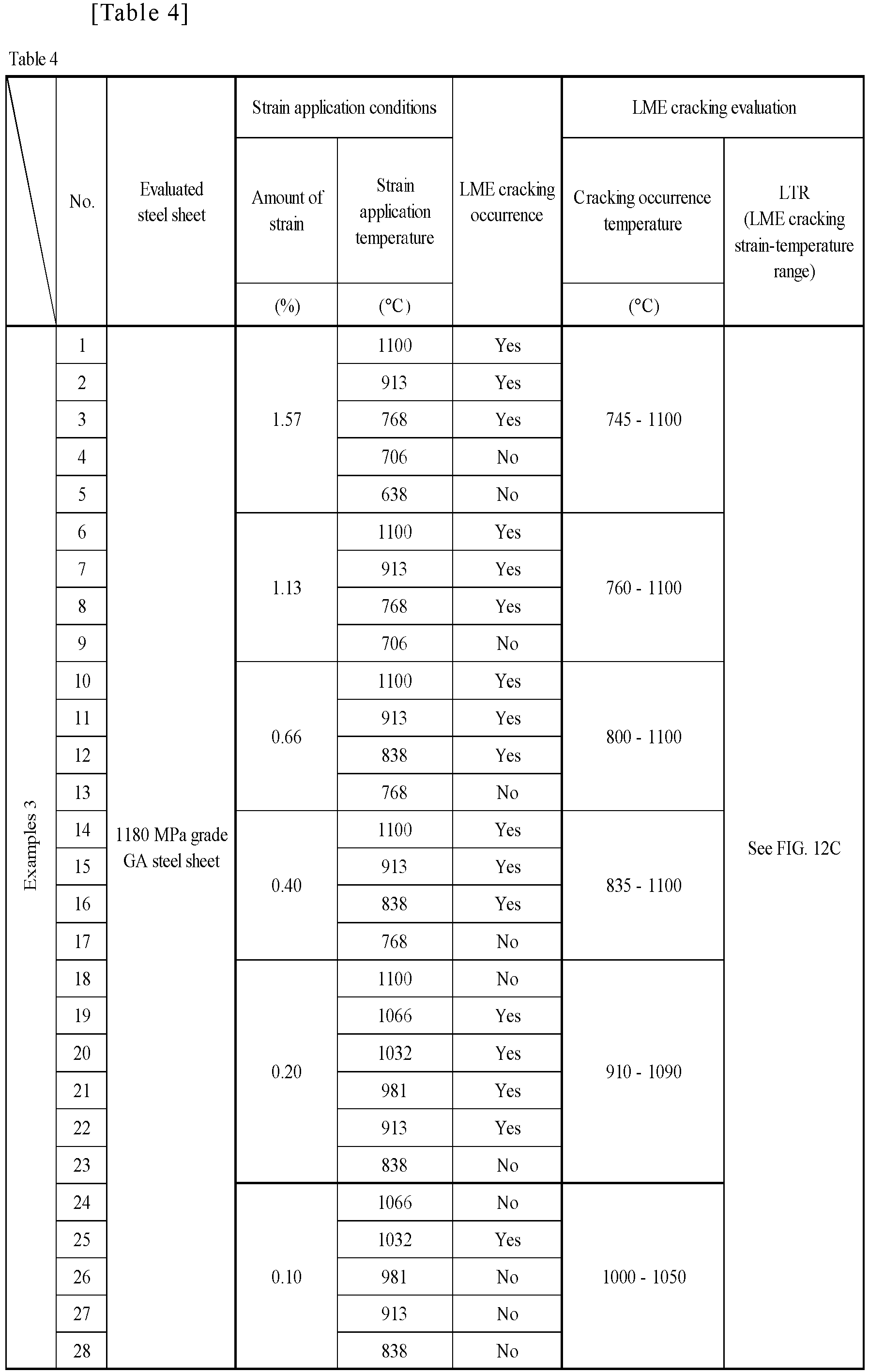

According to the Comparative Examples, no LME cracking was observed in the 780 MPa grade GA steel for any of the conventional evaluation methods, while for the 980 MPa grade GA steel, LME cracking occurred in the most severe sheet gap + electrode inclination angle method. Further, LME cracking occurred for the 1180 MPa grade GA steel in the sheet gap method, which confirms that susceptibility to LME cracking is qualitatively greater in the order of 780 MPa grade GA steel < 980 MPa grade GA steel < 1180 MPa grade GA steel, but this is not a quantitative evaluation and the extent of susceptibility cannot be checked.

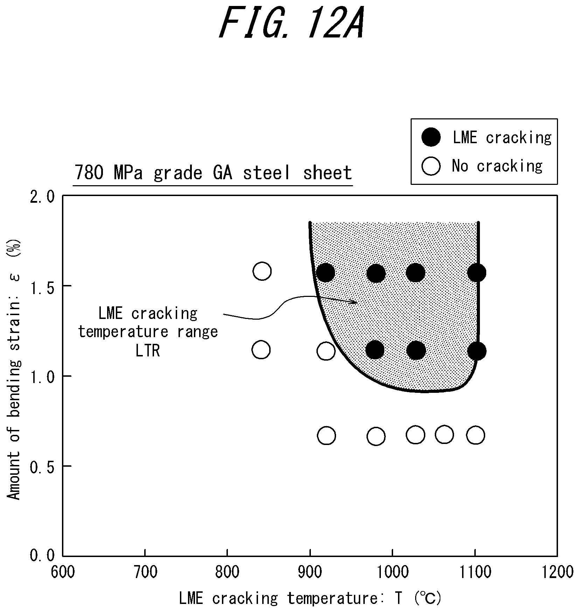

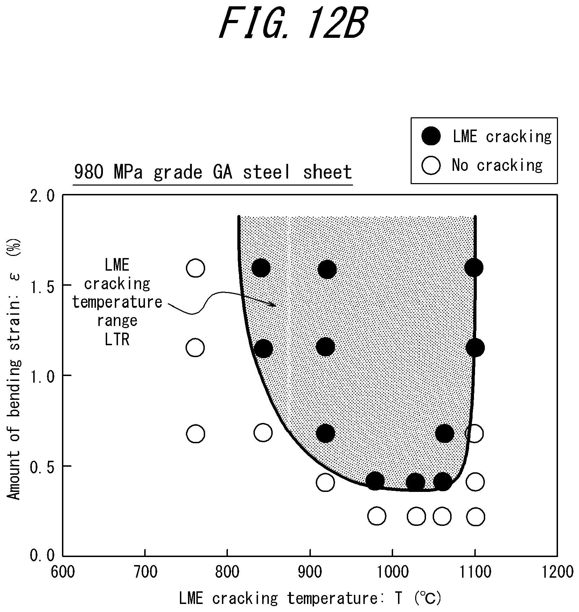

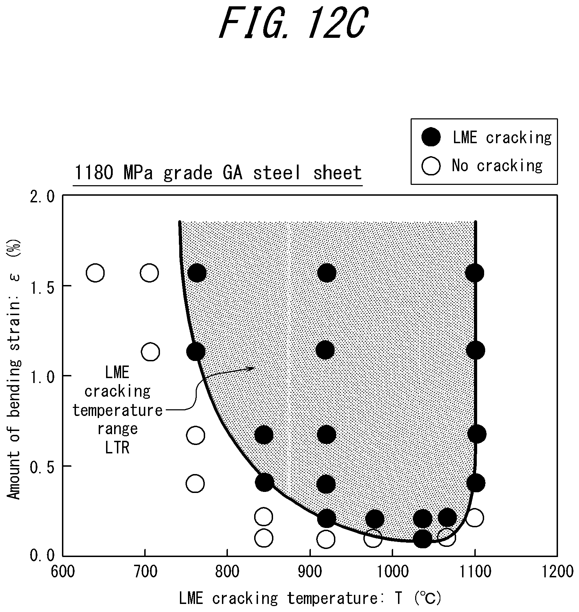

In contrast, according to the Examples, the upper limit of the amount of strain at which LME cracking does not occur is 0.66 % for 780 MPa grade GA steel and 0.20 % for 980 MPa grade GA steel. In the case of 1180 MPa grade GA steel, LME cracking occurs at an amount of strain of 0.10 % and the amount of strain at which LME cracking does not occur is less than 0.10 %. Thus, the evaluation for Examples 1 to 3 provides a quantitative understanding of susceptibility to LME cracking and also quantitatively and specifically confirms the risk of LME cracking for each material. The LME cracking strain-temperature ranges (LTR) for each steel sheet of Examples 1 to 3 are illustrated in FIG. 12A, FIG. 12B, and FIG. 12C, respectively. When illustrated as LTR, the critical amount of strain for LME cracking for each steel sheet may be confirmed more clearly: about 0.9 % for 780 MPa grade GA steel, about 0.3 % for 980 MPa grade GA steel, and about 0.1 % for 1180 MPa grade GA steel. Further, it can be seen that the temperature range where cracking occurs at the minimum amount of strain for all steel sheets is from 1000 °C to 1050 °C. As the applied strain increases, the temperature range at which cracking occurs expands, with the upper limit at 1100 °C and the lower limit at 750 °C.

Thus, according to Examples 1 to 3, it is possible to evaluate not only by the presence or absence of LME cracking, but also to evaluate a quantitative index that indicates the susceptibility to LME cracking of a material including a coated or plated layer.

### INDUSTRIAL APPLICABILITY

The method for evaluating susceptibility to liquid metal embrittlement cracking in a resistance spot welded portion of a steel sheet allows the susceptibility to liquid metal embrittlement cracking to be evaluated quantitatively and with high accuracy.

## Claims

1. A method for evaluating susceptibility to liquid metal embrittlement cracking in a resistance spot welded portion of a steel sheet, the method comprising:
a welding process of overlapping a first steel sheet and a second steel sheet, one or both of which are galvanized steel sheets, without an intervening gap to obtain a sheet combination, sandwiching the sheet combination between a pair of electrodes having an axial direction perpendicular to main surfaces of the first steel sheet and the second steel sheet, and applying current and pressure via the electrodes to perform resistance spot welding;
a strain applying process of applying one or both of bending strain and tensile strain controlled to a defined amount of strain to a welded portion of the second steel sheet at the same time as or after the end of the current application of the resistance spot welding;
an observation process of observing the welded portion of the second steel sheet to ascertain the presence or absence of liquid metal embrittlement cracking as an observation result; and
an evaluation process of evaluating susceptibility to liquid metal embrittlement cracking in the welded portion of the second steel sheet based on the observation result, wherein
a plurality of test pieces taken from the same type of steel sheet are used as the first steel sheet and a plurality of test pieces taken from the same type of steel sheet are used as the second steel sheet, and the series of processes consisting of the welding process, the strain applying process, and the observation process is performed a plurality of times,
the strain applying process is performed under a plurality of different conditions where one or both of a time at which strain is applied and the defined amount of strain are different, the plurality of different conditions consisting of a first set of conditions where liquid metal embrittlement cracking occurs in the welded portion of the second steel sheet and a second set of conditions where liquid metal embrittlement cracking does not occur in the welded portion of the second steel sheet, and
the evaluation process comprises:
identifying a location of occurrence of the liquid metal embrittlement cracking in the welded portion of the second steel sheet in at least one of the first set of conditions;
determining, among the plurality of conditions, (i) for the first set of conditions, temperature at the time of applying strain at the location of occurrence as a strain application temperature, and (ii) for the second set of conditions, temperature at the time of applying strain at a location corresponding to the location of occurrence as a strain application temperature;
determining a relationship between the strain application temperature and the defined amount of strain and the presence or absence of liquid metal embrittlement cracking, based on the observation results for the plurality of conditions; and
identifying, from the relationship, a range of amount of strain and temperature where liquid metal embrittlement cracking occurs in the welded portion of the second steel sheet;
determining, from the range, the minimum amount of strain at which liquid metal embrittlement cracking occurs in the welded portion of the second steel sheet; and
evaluating the susceptibility to liquid metal embrittlement cracking in the welded portion of the second steel sheet based on the minimum amount of strain, wherein it is evaluated that the higher the minimum amount of strain, the lower the susceptibility to liquid metal embrittlement cracking.

2. The method for evaluating susceptibility to liquid metal embrittlement cracking according to claim 1, wherein the welding process performed a plurality of times is performed under the same welding conditions.

3. The method for evaluating susceptibility to liquid metal embrittlement cracking according to claims 1 or 2, wherein the bending strain is applied in the strain applying process by applying bending deformation to the second steel sheet so that the second steel sheet has a certain curvature.

4. The method for evaluating susceptibility to liquid metal embrittlement cracking according to any one of claims 1 to 3, wherein the tensile strain is applied in the strain applying process by applying tensile deformation to the second steel sheet.

5. The method for evaluating susceptibility to liquid metal embrittlement cracking according to any one of claims 1 to 4, wherein the observation process comprises separating the first steel sheet from the second steel sheet and observing the welded portion of the second steel sheet from a main surface side where the second steel sheet was welded to the first steel sheet.

## Patentansprüche

1. Verfahren zur Bewertung der Anfälligkeit für Flüssigmetallversprödungsrisse in einem widerstandspunktgeschweißten Teil eines Stahlblechs, wobei das Verfahren umfasst:
einen Schweißprozess, bei dem ein erstes Stahlblech und ein zweites Stahlblech, von denen eines oder beide verzinkte Stahlbleche sind, spaltfrei überlappt werden, um eine Blechkombination zu erhalten, die Blechkombination zwischen ein Elektrodenpaar eingespannt wird, das eine Achsrichtung senkrecht zu den Hauptflächen des ersten Stahlblechs und des zweiten Stahlblechs aufweist, und über die Elektroden Strom und Druck angelegt werden, um Widerstandspunktschweißen durchzuführen;
einen Dehnungsanlegungsprozess, bei dem eine oder beide von Biegedehnung und Zugdehnung gesteuert auf einen definierten Dehnungsbetrag gleichzeitig mit oder nach Beendigung der Stromzufuhr des Widerstandspunktschweißens an dem Schweißteil des zweiten Stahlblechs angelegt werden;
einen Beobachtungsprozess des Beobachtens des Schweißteils des zweiten Stahlblechs, um das Vorliegen oder Nichtvorliegen von Flüssigmetallversprödungsrissen als Beobachtungsergebnis festzustellen; und
einen Bewertungsprozess zum Bewerten der Anfälligkeit für Flüssigmetallversprödungsrisse im Schweißteil des zweiten Stahlblechs auf Grundlage des Beobachtungsergebnisses, wobei
eine Vielzahl von Probekörpern, die aus demselben Typ von Stahlblech entnommen sind, als erstes Stahlblech verwendet werden und eine Vielzahl von Probekörpern, die aus demselben Typ von Stahlblech entnommen sind, als zweites Stahlblech verwendet werden, und die aus dem Schweißprozess, dem Dehnungsanlegungsprozess und dem Beobachtungsprozess bestehende Abfolge von Prozessen mehrfach durchgeführt wird,
der Dehnungsanlegungsprozess unter eine Vielzahl von unterschiedlichen Bedingungen durchgeführt wird, bei denen eines oder beide von einem Zeitpunkt der Dehnungsanlegung und der definierte Dehnungsbetrag unterschiedlich sind, wobei die Vielzahl von unterschiedlichen Bedingungen aus einem ersten Satz von Bedingungen besteht, bei dem Flüssigmetallversprödungsrisse im Schweißteil des zweiten Stahlblechs auftreten, und einem zweiten Satz von Bedingungen, bei dem Flüssigmetallversprödungsrisse im Schweißteil des zweiten Stahlblechs nicht auftreten, und
der Bewertungsprozess umfasst:
Ermitteln einer Stelle des Auftretens der Flüssigmetallversprödungsrisse im Schweißteil des zweiten Stahlblechs in zumindest einem des ersten Satzes von Bedingungen;
Bestimmen, unter der Vielzahl von Bedingungen, (i) für den ersten Satz von Bedingungen, der Temperatur zum Zeitpunkt der Dehnungsanlegung an der Stelle des Auftretens als Dehnungsanlegetemperatur und (ii) für den zweiten Satz von Bedingungen, der Temperatur zum Zeitpunkt der Dehnungsanlegung einer der Stelle des Auftretens entsprechenden Stelle als Dehnungsanlegetemperatur;
Bestimmen einer Beziehung zwischen der Dehnungsanlegetemperatur und dem definierten Dehnungsbetrag und dem Vorliegen oder Nichtvorliegen von Flüssigmetallversprödungsrissen auf Grundlage der Beobachtungsergebnisse für die Vielzahl von Bedingungen; und
Ermitteln, aus der Beziehung, eines Bereichs von Dehnungsbetrag und Temperatur, in dem Flüssigmetallversprödungsrisse im Schweißteil des zweiten Stahlblechs auftreten;
Bestimmen, aus dem Bereich, des Mindestdehnungsbetrags, bei dem Flüssigmetallversprödungsrisse im Schweißteil des zweiten Stahlblechs auftreten; und
Bewerten der Anfälligkeit für Flüssigmetallversprödungsrisse im Schweißteil des zweiten Stahlblechs auf Grundlage des Mindestdehnungsbetrags, wobei bewertet wird, dass je größer der Mindestdehnungsbetrag, desto geringer die Anfälligkeit für Flüssigmetallversprödungsrisse.

2. Verfahren zur Bewertung der Anfälligkeit für Flüssigmetallversprödungsrisse nach Anspruch 1, wobei der mehrfach durchgeführte Schweißprozess unter denselben Schweißbedingungen durchgeführt wird.

3. Verfahren zur Bewertung der Anfälligkeit für Flüssigmetallversprödungsrisse nach Anspruch 1 oder 2, wobei die Biegedehnung im Dehnungsanlegungsprozess durch Anlegen einer Biegeverformung an dem zweiten Stahlblech angelegt wird, sodass das zweite Stahlblech eine bestimmte Krümmung aufweist.

4. Verfahren zur Bewertung der Anfälligkeit für Flüssigmetallversprödungsrisse nach einem der Ansprüche 1 bis 3, wobei die Zugdehnung im Dehnungsanlegungsprozess durch Anlegen einer Zugverformung an dem zweiten Stahlblech angelegt wird.

5. Verfahren zur Bewertung der Anfälligkeit für Flüssigmetallversprödungsrisse nach einem der Ansprüche 1 bis 4, wobei der Beobachtungsprozess das Trennen des ersten Stahlblechs von dem zweiten Stahlblech und das Beobachten des Schweißteils des zweiten Stahlblechs von der Hauptflächenseite, an der das zweite Stahlblech an das erste Stahlblech geschweißt wurde, umfasst.

## Revendications

1. Procédé d'évaluation de la susceptibilité à la fissuration par fragilisation par métal liquide dans une partie soudée par points par résistance d'une plaque d'acier, le procédé comprenant :
un procédé de soudage consistant à superposer une première plaque d'acier et une seconde plaque d'acier, dont l'une ou les deux sont des plaques d'acier zinguées, sans espace afin d'obtenir une superposition de plaques, intercalant la superposition de plaques entre une paire d'électrodes présentant une direction axiale perpendiculaire aux surfaces principales de la première plaque d'acier et de la seconde plaque d'acier, et à appliquer, via les électrodes, un courant et une pression afin de réaliser un soudage par points par résistance ;
un procédé d'application de contrainte consistant à appliquer une contrainte de flexion ou de traction, ou les deux, contrôlée(s) à un niveau de contrainte défini, à une partie soudée de la seconde plaque d'acier simultanément à ou après la fin de l'énergisation du soudage par points par résistance ;
un procédé d'observation consistant à observer la partie soudée de la seconde plaque d'acier afin de constater la présence ou l'absence de fissuration par fragilisation par métal liquide à titre de résultat d'observation ; et
un procédé d'évaluation consistant à évaluer la susceptibilité à la fissuration par fragilisation par métal liquide dans la partie soudée de la seconde plaque d'acier sur la base du résultat d'observation, dans lequel
une pluralité de pièces d'essai prélevées sur le même type de plaque d'acier sont utilisées comme première plaque d'acier et une pluralité de pièces d'essai prélevées sur le même type de plaque d'acier sont utilisées comme seconde plaque d'acier, et la série de procédés constituée du procédé de soudage, du procédé d'application de contrainte et du procédé d'observation est réalisée une pluralité de fois,
le procédé d'application de contrainte est réalisé sous une pluralité de conditions différentes où l'un ou les deux du moment auquel la contrainte est appliquée et du niveau de contrainte défini sont différents, la pluralité de conditions différentes consistant en un premier ensemble de conditions où une fissuration par fragilisation par métal liquide se produit dans la partie soudée de la seconde plaque d'acier et un second ensemble de conditions où une fissuration par fragilisation par métal liquide ne se produit pas dans la partie soudée de la seconde plaque d'acier, et
le procédé d'évaluation comprend :
l'identification d'un site d'apparition de la fissuration par fragilisation par métal liquide dans la partie soudée de la seconde plaque d'acier dans au moins une des conditions du premier ensemble de conditions ;
la détermination, parmi la pluralité de conditions, (i) pour le premier ensemble de conditions, de la température au moment de l'application de la contrainte au site d'apparition en tant que température d'application de la contrainte, et (ii) pour le second ensemble de conditions, de la température au moment de l'application de la contrainte à un emplacement correspondant au site d'apparition en tant que température d'application de la contrainte ;
la détermination, sur la base des résultats d'observation pour la pluralité de conditions, d'une relation entre la température d'application de la contrainte et le niveau de contrainte défini et la présence ou l'absence de fissuration par fragilisation par métal liquide ; et
l'identification, à partir de la relation, d'une plage de niveau de contrainte et de la température où une fissuration par fragilisation par métal liquide se produit dans la partie soudée de la seconde plaque d'acier ;
la détermination, à partir de la plage, du niveau de contrainte minimal auquel une fissuration par fragilisation par métal liquide se produit dans la partie soudée de la seconde plaque d'acier ; et
l'évaluation de la susceptibilité à la fissuration par fragilisation par métal liquide dans la partie soudée de la seconde plaque d'acier sur la base du niveau de contrainte minimal, dans laquelle il est évalué que plus le niveau de contrainte minimal est élevé, plus la susceptibilité à la fissuration par fragilisation par métal liquide est faible.

2. Procédé d'évaluation de la susceptibilité à la fissuration par fragilisation par métal liquide selon la revendication 1, dans lequel le procédé de soudage réalisé une pluralité de fois est réalisé dans les mêmes conditions de soudage.

3. Procédé d'évaluation de la susceptibilité à la fissuration par fragilisation par métal liquide selon la revendication 1 ou la revendication 2, dans lequel la contrainte de flexion est appliquée lors du procédé d'application de contrainte en appliquant une déformation de flexion à la seconde plaque d'acier de sorte que la seconde plaque d'acier présente une certaine courbure.

4. Procédé d'évaluation de la susceptibilité à la fissuration par fragilisation par métal liquide selon l'une quelconque des revendications 1 à 3, dans lequel la contrainte de traction est appliquée lors du procédé d'application de contrainte en appliquant une déformation de traction à la seconde plaque d'acier.

5. Procédé d'évaluation de la susceptibilité à la fissuration par fragilisation par métal liquide selon l'une quelconque des revendications 1 à 4, dans lequel le procédé d'observation comprend la séparation de la première plaque d'acier de la seconde plaque d'acier et l'observation de la partie soudée de la seconde plaque d'acier du côté de la surface principale par laquelle la seconde plaque d'acier a été soudée à la première plaque d'acier.
